# EUROPEAN PATENT APPLICATION

(11) **EP 3 741 863 A1**
(43) Date of publication of application: **25.11.2020**
(21) Application number: 19175322.7
(22) Date of filing: 20.05.2019
(51) Int. Cl.: C12P 7/04, B01D 53/62, B01D 53/96, B01D 61/44, C02F 1/469, C12P 7/06, C12P 7/40, C12P 7/54

(54) **REGENERATING AND UTILIZING CARBON DIOXIDE**

(71) Applicant: Evonik Operations GmbH, 45128 Essen (DE)
(72) Inventor: HAAS, Thomas, 48161 Münster (DE)
(74) Representative: Evonik Patent Association

(57) **Abstract**

The present invention relates to a method for production of organic acid and/or alcohol comprising the steps:
a) providing a basic, aqueous medium containing captured carbon dioxide in the form of hydrogencarbonate-anions and/or carbonate-anions;
b) feeding the basic, aqueous medium containing the captured carbon dioxide into a bipolar membrane electrodialysis unit comprising
an anode,
a cathode,
an ion-selective anion-exchange membrane, and
two water-dissociating bipolar membranes,
capable of providing for an acidic compartment between the water-dissociating bipolar membrane on the anode side and the ion-selective anion-exchange membrane and for a basic compartment between the water-dissociating bipolar membrane on the cathode side and the ion-selective anion-exchange membrane;
c) regenerating the captured carbon dioxide to regenerated carbon dioxide by transporting the captured carbon dioxide in the form of hydrogencarbonate-anions and/or carbonate-anions across the anion-exchange membrane from the basic compartment into the acidic compartment in acidic, aqueous solution;
d) contacting the regenerated carbon dioxide and hydrogen with at least one acetogenic cell in an aqueous production medium under suitable conditions to produce at least one organic acid and/or alcohol from the regenerated carbon dioxide; and optionally
e) recovering the organic acid and/or alcohol.

## Description

### Field of the invention

The present invention relates to a method for production of organic acid and/or alcohol comprising the steps:
a) providing a basic, aqueous medium containing captured carbon dioxide in the form of hydrogencarbonate-anions and/or carbonate-anions;
b) feeding the basic, aqueous medium containing the captured carbon dioxide into a bipolar membrane electrodialysis unit comprising
   an anode,
   a cathode,
   an ion-selective anion-exchange membrane, and
   two water-dissociating bipolar membranes,
   capable of providing for an acidic compartment between the water-dissociating bipolar membrane on the anode side and the ion-selective anion-exchange membrane and for a basic compartment between the water-dissociating bipolar membrane on the cathode side and the ion-selective anion-exchange membrane;
c) regenerating the captured carbon dioxide to regenerated carbon dioxide by transporting the captured carbon dioxide in the form of hydrogencarbonate-anions and/or carbonate-anions across the anion-exchange membrane from the basic compartment into the acidic compartment in acidic, aqueous solution;
d) contacting the regenerated carbon dioxide and hydrogen with at least one acetogenic cell in an aqueous production medium under suitable conditions to produce at least one organic acid and/or alcohol from the regenerated carbon dioxide; and optionally
e) recovering the organic acid and/or alcohol.

### Prior art

Carbon dioxide (CO₂) is a greenhouse gas that is collecting in the atmosphere and causing global warming problems and unwanted climate changes. Most industrial processes that are being carried out internationally release inadvertently small amounts of CO₂ in their exhaust fumes. This CO₂ is being accumulated in the environment and causing an increase in temperature that is leading to uncontrollable climate changes such as melting of the polar ice caps, increase in sea-level globally, floods and the like.

There is thus a need to stabilise atmospheric CO₂ to reduce these problems. One way to do so would be to reduce emissions by increasing the efficiency of combustion processes, as in fossil fuel-based power plants or motor vehicles with internal combustion engines. However, this is an arduous task taking into the account the increase in the number of cars in the world and the increase in the emissions from burning fossil fuels. Another way is to substitute fossil fuels in carbon-based processes with renewable energy or low-carbon fuels. However, this would reduce emissions but increase fuel costs. Accordingly, there is a need in the art to direct capture CO₂ from the atmosphere and to reduce and stabilise the amount of CO₂ in ambient air at 350 to 440 parts per million by volume (ppm).

There is also a need to store this captured CO₂ in a form where it may be used as an alternative to fossil fuels in view of the depleting amount of fossil fuels available on earth. There are currently numerous technologies and materials for capturing CO₂ from ambient air discussed in the literature using absorption/adsorption processes with inorganic chemisorbents, amines/imines, zeolites, anionic exchange resins and the like. Processes applied for carbon capture and storage cannot be directly transferred to CO₂ separation from the air due to the humidity of the air, very low CO₂ concentrations and the necessity to process close to ambient temperature and pressure conditions (Goeppert A., (2012), Energy Environ Sci., 5: 7833). Further, in Krekel, D., (2018), Applied Energy, 218:361-381 it has been concluded that the results reported show that CO₂ separation from ambient air will not be able to play a vital role in the abatement of the climate change problem until 2050 due to its strong technological and economic drawbacks.

In Sanz-Perez E., (2016) Chem. Rev., 116: 11840-11876, a historical review of different Direct Air Capture (DAC) techniques are disclosed. In particular, different chemical sorbents that are cycled through sorption and desorption cycles for CO₂ removal from ultra-dilute gases such as air are disclosed in this paper. However, these methods disclosed are not competitive especially since desorbing of CO₂ costs a lot of energy and money. There is thus a need in the art for directly capturing CO₂ from ambient air without using desorbing of CO₂.

More in particular, there is a need in the art to not only directly capture CO₂ from ambient air but also to store the CO₂ so that the stored CO₂ may be used as an alternative source for higher chemicals.

### Description of the invention

The present invention attempts to solve the problems above by providing a method for production of organic acid and/or alcohol from carbon dioxide and by providing a means for production of organic acid and/or alcohol comprising a bipolar membrane electrodialysis unit and a fermenter. In particular, the present invention captures the carbon dioxide, converts the captured carbon dioxide to a regenerated carbon dioxide, which can be directly used for the production of an organic acid and/or alcohol using at least one microorganism. This method provides the advantages of being capable of reducing the carbon dioxide concentration in the air as well as the method provides a use for the carbon dioxide which is stored.

The present invention relates to a method for production of organic acid and/or alcohol comprising the steps:
a) providing a basic, aqueous medium containing captured carbon dioxide in the form of hydrogencarbonate-anions and/or carbonate-anions;
b) feeding the basic, aqueous medium containing the captured carbon dioxide into a bipolar membrane electrodialysis unit comprising
   an anode,
   a cathode,
   an ion-selective anion-exchange membrane, and
   two water-dissociating bipolar membranes,
   capable of providing for an acidic compartment between the water-dissociating bipolar membrane on the anode side and the ion-selective anion-exchange membrane and for a basic compartment between the water-dissociating bipolar membrane on the cathode side and the ion-selective anion-exchange membrane;
c) regenerating the captured carbon dioxide to regenerated carbon dioxide by transporting the captured carbon dioxide in the form of hydrogencarbonate-anions and/or carbonate-anions across the anion-exchange membrane from the basic compartment into the acidic compartment in acidic, aqueous solution;
d) contacting the regenerated carbon dioxide and hydrogen with at least one acetogenic cell in an aqueous production medium under suitable conditions to produce at least one organic acid and/or alcohol from the regenerated carbon dioxide; and optionally
e) recovering the organic acid and/or alcohol.

The term "basic medium" in the context of the instant invention refers to a medium having a pH of bigger than 7.

The term "acidic medium" in the context of the instant invention refers to a medium having a pH of smaller than 7.

The term "captured carbon dioxide" in the context of the instant invention refers to carbon dioxide contained in a basic, aqueous medium either in the form of hydrogencarbonate-anions and/or carbonate-anions and/or in the form of carbon dioxide being physically dissolved in a basic, aqueous medium.

The term "regenerated carbon dioxide" in the context of the instant invention refers to carbon dioxide contained in an acidic, aqueous medium either in the form of hydrogencarbonate-anions and/or carbonate-anions and/or in the form of carbon dioxide being physically dissolved in an acidic, aqueous medium.

The term "aqueous medium" in the context of the instant invention refers to a medium having a water content of at least 50 wt.-% referring to the total medium.

The "pH" in connection with the present invention is defined as the value which is measured for the relevant composition at 25°C after stirring for 5 minutes using a calibrated pH electrode in accordance with ISO 4319 (1977).

Unless otherwise stated, all percentages (%) given are percentages by weight.

The provision of the basic, aqueous medium containing captured carbon dioxide in the form of hydrogencarbonate-anions and/or carbonate-anions can be easily achieved by collecting seawater: natural seawater is slightly basic and thus is capable of naturally capturing carbon dioxide from the ambient air. Thus taking sea water and feeding it into the process of the instant invention is an advantageous method step a) according to the instant invention

In a preferred method according to the instant invention step a) comprises
providing a mixture of gases comprising carbon dioxide and contacting the mixture of gases with a basic, aqueous medium containing at least one base, thereby capturing the carbon dioxide, which is contained in the basic, aqueous medium in the form of hydrogencarbonate- and/or carbonate-anions.

It is a preferred method according to the instant invention, that the mixture of gases comprises carbon dioxide at a concentration of 350ppm-450ppm, preferably the mixture of gases is air.

In a preferred method according to the instant invention the basic, aqueous medium in step a) contains a base selected from the group of hydroxides and carbonates, preferably of alkaline and earth alkaline hydroxides and carbonates, preferably from the group of sodium hydroxide, potassium hydroxide and calcium hydroxide.

It is a preferred method according to the instant invention, that in step a) the basic, aqueous medium is sprayed through a nozzle into a stream of the mixture of gases comprising carbon dioxide.

In step b) of the instant invention the basic, aqueous medium containing the captured carbon dioxide is fed into a bipolar membrane electrodialysis unit.

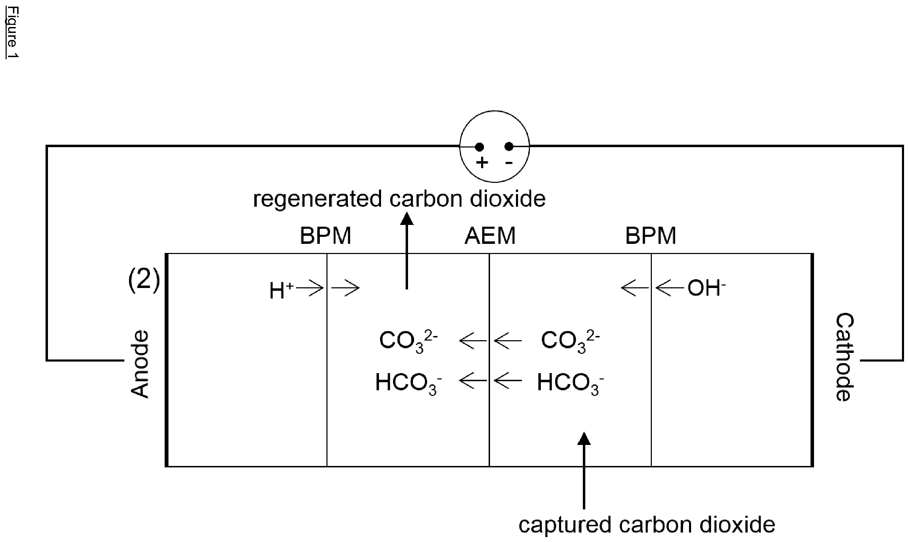

Figure 1 shows an example of such a bipolar membrane electrodialysis unit: The carbon dioxide exists in solution as negatively charged hydrogencarbonate- and/or carbonate-anions. This solution is fed into a a bipolar membrane electrodialysis (BPMED) unit, which separates the Hydrogencarbonate/carbonate solution into an acid and a base by applying a voltage across an alternating stack of ion-selective anion-exchange membranes (AEMs) and water-dissociating bipolar membranes (BPMs), and transporting carbon dioxide as hydrogencarbonate- and/or carbonate-anions into the acidic compartment.

The acidic solution can convert the transported hydrogencarbonate- and/or carbonate-anions into carbon dioxide, and the low solubility of total dissolved carbon dioxide in the acidic solution can results in carbon dioxide gas evolution, depending on carbon dioxide concentration, temperature and pressure applied.

It is a preferred method of the instant invention, when in step b) the bipolar membrane electrodialysis unit comprises an alternating stack of ion-selective anion-exchange membranes and water-dissociating bipolar membranes of an amount of from 3 to 20 membranes.

Suited membranes that can be used in the bipolar membrane electrodialysis unit of the instant invention are readily commercially available, for example as ion-selective anion-exchange membranes Neosepta AHA and as water-dissociating bipolar membranes Neosepta BP-1E, both nmanufactured by Ameridia Corp. can be used.

Preferably the the bipolar membrane electrodialysis unit used in the process of the instant invention further comprises an ion-selective cation exchange membrane, that separates the acidic compartment from the anode and the basic compartment from the cathode. Preferably the ion-selective cation exchange membrane is Neosepta C66-10F supplied by Ameridia Corp.

It is a preferred method according to the instant invention, that the pH values within the bipolar membrane electrodialysis unit of the instant invention are kept within constant ranges: preferably the pH of the acidic compartment is maintained in the range of from 1.5 to 7.0, preferably of from 3.0 to 7.0, most preferably from 4.5 to 6.0; preferably the pH of the basic compartment is maintained in the range of from 7.1 to 12.0, preferably of from 8.0 to 11.5, most preferably from 9.5 to 11.0.

The pH values within the bipolar membrane electrodialysis unit of the instant invention can be kept within constant ranges by adding an acid or a base to the aqueous solutions in the bipolar membrane electrodialysis unit. Usually acid is needed, as the pH in both compartments tends to rise rather than to fall during the method according to the instant invention.

In step c) the captured carbon dioxide is regenerated. In a preferred method according to the instant invention in step c) an amperage of 5 A to 20 A, preferably 5 A to 15A most preferred 5 A to 10 A is applied to the bipolar membrane electrodialysis unit. Preferably in the method according to the instant invention in step c) a voltage of 5 V to 30V, preferably of 10 V to 25V, most preferably of 10 V to 20V is applied to the bipolar membrane electrodialysis unit.

In a preferred method according to the instant invention in step c) the acidic, aqueous solution in the acidic compartment is kept at a temperature within the range of from 1.0 °C to 15 °C, preferably from 6.0°C to 12 °C, even more preferred from 8 °C to 10 °C. This has the technical effect that the captured carbon dioxide can be transported at higher rates.

In a preferred method according to the instant invention in step c) the acidic, aqueous solution in the acidic compartment contains an organic extracting medium, which is used in step e) for the recovering of the organic acid and/or alcohol from step d); for details see below, also for the preferred organic extracting media used in this aspect. This also has the technical effect that the captured carbon dioxide can be transported at higher rates.

In step d) the regenerated carbon dioxide is converted to an organic acid and/or alcohol by acetogenic bacteria.

The term "acetogenic bacteria" as used herein refers to a microorganism which is able to perform the Wood-Ljungdahl pathway and thus is able to convert CO, CO₂ and/or hydrogen to acetate. These microorganisms include microorganisms which in their wild-type form do not have a Wood-Ljungdahl pathway, but have acquired this trait as a result of genetic modification. Such microorganisms include but are not limited to E. coli cells. These microorganisms may be also known as carboxydotrophic bacteria. Currently, 21 different genera of the acetogenic bacteria are known in the art (Drake et al., 2006), and these may also include some clostridia (Drake & Kusel, 2005). These bacteria are able to use carbon dioxide or carbon monoxide as a carbon source with hydrogen as an energy source (Wood, 1991). Further, alcohols, aldehydes, carboxylic acids as well as numerous hexoses may also be used as a carbon source (Drake et al., 2004). The reductive pathway that leads to the formation of acetate is referred to as acetyl-CoA or Wood-Ljungdahl pathway.

In particular, the acetogenic bacteria may be selected from the group consisting of *Acetoanaerobium notera (ATCC 35199), Acetonema longum (DSM 6540), Acetobacterium carbinolicum (DSM 2925), Acetobacterium malicum (DSM 4132), Acetobacterium species no. 446 (*Morinaga et al., 1990, J. Biotechnol., Vol. 14, p. 187-194*),Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950, formerly Ruminococcus productus, formerly Peptostreptococcus productus), Butyribacterium methylotrophicum (DSM 3468), Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium ljungdahlii (DSM 13528), Clostridium ljungdahlii C-01 (ATCC 55988), Clostridium ljungdahlii ERI-2 (ATCC 55380), Clostridium ljungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757), Clostridium species ATCC 29797 (*Schmidt et al., 1986, Chem. Eng. Commun., Vol. 45, p. 61-73*), Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM 2834), Moorella sp. HUC22-1 (*Sakai et al., 2004, Biotechnol. Let., Vol. 29, p. 1607-1612*), Moorella thermoacetica (DSM 521, formerly Clostridium thermoaceticum), Moorella thermoautotrophica (DSM 1974), Oxobacter pfennigii (DSM 322), Sporomusa aerivorans (DSM 13326), Sporomusa ovata (DSM 2662), Sporomusa silvacetica (DSM 10669), Sporomusa sphaeroides (DSM 2875), Sporomusa termitida (DSM 4440) and Thermoanaerobacter kivui (DSM 2030,* formerly *Acetogenium kivui*), the strains in brackets being preferred strains.

More in particular, the acetogenic cell may be selected from the *Clostridium* family. Even more in particular, the acetogenic cell used according to any aspect of the present invention may be selected from the group consisting of *Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium ljungdahlii (DSM 13528), Clostridium ljungdahlii C-01 (ATCC 55988), Clostridium ljungdahlii ERI-2 (ATCC 55380), Clostridium ljungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757),* and *Clostridium species ATCC 29797*), the strains in brackets being preferred strains.

In particular, the strain ATCC BAA-624 of *Clostridium carboxidivorans* may be used. Even more in particular, the bacterial strain labelled "P7" and "P11" of *Clostridium carboxidivorans* as described for example in U.S. 2007/0275447 and U.S. 2008/0057554 may be used.

Another particularly suitable bacterium may be *Clostridium ljungdahlii.* In particular, strains selected from the group consisting of *Clostridium ljungdahlii* PETC, *Clostridium ljungdahlii* ERI2, *Clostridium ljungdahlii* COL and *Clostridium ljungdahlii* O-52 may be used in the conversion of synthesis gas to hexanoic acid. These strains for example are described in WO 98/00558, WO 00/68407, ATCC 49587, ATCC 55988 and ATCC 55989.

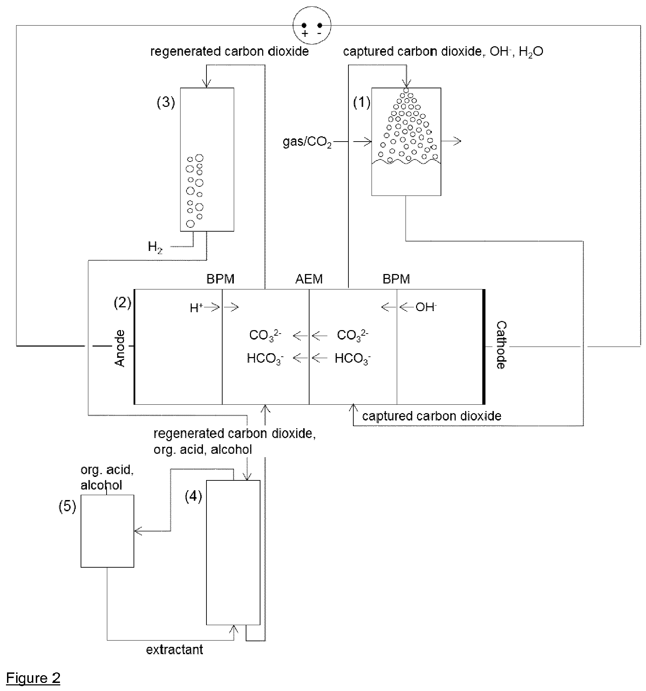

It is a preferred method according to the instant invention, that in step d) the contacting takes place in a fermenter comprising the at least one acetogenic cell, the aqueous production medium, a means of receiving a hydrogen stream, and a means of receiving the recovered carbon dioxide. The fermenter is schematically depicted in (3) of figure 2.

In some examples, acetogenic cells capable of producing the organic acid and/or alcohol may be cultivated with any aqueous production media, substrates, conditions, and processes generally known in the art for culturing bacteria. This allows for the organic acid and/or alcohol to be produced using a biotechnological method. Depending on the microorganism that is used for organic acid and/or alcohol production, appropriate growth medium, pH, temperature, agitation rate, inoculum level, and/or aerobic, microaerobic, or anaerobic conditions are varied. A skilled person would understand the other conditions necessary to carry out the method according to any aspect of the present invention. In particular, the conditions in the fermenter may be varied depending on the microorganisms used. The varying of the conditions to be suitable for the optimal functioning of the microorganisms is within the knowledge of a skilled person.

In one example, the step d) according to any aspect of the present invention may be carried out wherein in step d) the pH of the aqueous production medium is in the range between 4.0 and 6.9, preferably between 5.0 and 7.0, most preferably between 5.0 and 6.5. The pressure may be between 0.9 and 10 bar. The microorganisms may be cultured at a temperature ranging from about 20 °C to about 80 °C, preferably ranging from about 25 °C to about 40 °C. In one example, the microorganism may be cultured at 37 °C.

In a preferred method of the instant invention the pH values within the bipolar membrane electrodialysis unit of the instant invention of the acidic compartment in step c) is maintained in the range of from 5.0 to 6.5 and in step d) the pH of the aqueous production medium is in the range between 5.0 and 6.5. This has the advantage, that further pH adjustments between the two steps are not necessary. Furthermore within this pH-range the regenerated carbon dioxide will remain dissolved in the acidic, aqueous medium without the need of applying high pressure; thus preferably in this context steps c) and d) of the method according to the instant invention are carried out within a pressure range of 0.9 to 1.2 bar.

In some examples, for the growth of the microorganism and for its production of organic acid and/or alcohol, the the aqueous production medium may comprise any nutrients, ingredients, and/or supplements suitable for growing the microorganism or for promoting the production of the organic acid and/or alcohol. In particular, the the aqueous production medium may comprise at least one of the following: carbon sources, nitrogen sources, such as an ammonium salt, yeast extract, or peptone; minerals; salts; cofactors; buffering agents; vitamins; and any other components and/or extracts that may promote the growth of the bacteria. The the aqueous production medium to be used must be suitable for the requirements of the particular strains. Descriptions of aqueous production media for various microorganisms are given in "Manual of Methods for General Bacteriology".

A skilled person would thus be able to determine the suitable conditions that would allow for the production of at least one organic acid and/or alcohol from the regenerated carbon dioxide.

In one example according to any aspect of the present invention, the acetogenic cell in step d) converts the the regenerated carbon dioxide to at least one alcohol, at least one organic acid or a mixture of alcohol and organic acid. In particular, the organic acid may be selected from the group consisting of acetic acid, butyric acid, hexanoic acid and mixture thereof. The alcohol may be selected from the group consisting of ethanol, butanol, hexanol and mixtures thereof. In one example, a combination of both an alcohol and an organic acid is formed. Most preferably the alcohol is ethanol and the organic acid acetic acid.

Step e) according to any aspect of the present invention involves the recovering of the organic acid and/or alcohol from step d).

The organic acid and/or alcohol contained in the aqueous production medium preferably is contacted in step e) with an organic extracting medium and results in the formation of two phases. As organic extracting medium in step e) the organic extracting medium preferably comprises:
at least one alkyl-phosphine oxide and at least one alkane comprising at least 12 carbon atoms; or
at least one trialkylamine and at least one alkane comprising at least 12 carbon atoms.

The organic extracting medium according to any aspect of the present invention may efficiently extract the organic acid and/or alcohol into the organic extracting medium. This organic extracting medium of a mixture of alkyl-phosphine oxide or trialkylamine and at least one alkane may be considered suitable in the method according to any aspect of the present invention as the mixture works efficiently in extracting the desired organic acid and/or alcohol in the presence of the aqueous production medium. In particular, the mixture of alkyl-phosphine oxide or trialkylamine and at least one alkane may be considered to work better than any method currently known in the art for extraction organic acid and/or alcohol as it does not require any special equipment to be carried out and it is relatively easy to perform with a high product yield. Further, the organic extracting medium according to any aspect of the present invention is also not toxic for the acetogenic cell of step d).

The alkane may comprise at least 12 carbon atoms. In particular, the alkane may comprise 12-18 carbon atoms. In one example, the alkane may be selected from the group consisting of dodecane, tridecane, tetradecane, pentadecane, hexadecane, heptadecane and octadecane. In a further example, the organic extracting medium may comprise a mixture of alkanes.

Alkyl-phosphine oxides have a general formula of OPX₃, where X is an alkyl. Suitable alkyl phosphine oxides according to any aspect of the present invention include an alkyl group composed of a linear, branched or cyclic hydrocarbon, the hydrocarbon composed of from 1 to about 100 carbon atoms and from 1 to about 200 hydrogen atoms. In particular, "alkyl" as used in reference to alkyl phosphine oxide according to any aspect of the present invention can refer to a hydrocarbon group having 1 to 20 carbon atoms, frequently between 4 and 15 carbon atoms, or between 6 and 12 carbon atoms, and which can be composed of straight chains, cyclics, branched chains, or mixtures of these. The alkyl phosphine oxide may have from one to three alkyl groups on each phosphorus atom. In one example, the alkyl phosphine oxide has three alkyl groups on P. In some examples, the alkyl group may comprise an oxygen atom in place of one carbon of a C4-C15 or a C6-C12 alkyl group, provided the oxygen atom is not attached to P of the alkyl phosphine oxide. Typically, the alkyl phosphine oxide is selected from the group consisting of tri-octylphosphine oxide, tri-butylphosphine oxide, hexyl-phosphine oxide, octylphosphine oxide and mixtures thereof. Even more in particular, the alkyl phosphine oxide may be tri-octylphosphine oxide (TOPO).

Trialkylamines are organic-chemical compounds derived from ammonia (NH₃), whose three hydrogen atoms are replaced by alkyl radicals. Examples of trialkylamines are dimethylethylamine, methyldiethylamine, triethylamine, dimethyl-n-propylamine, dimethyl-i-propylamine, methyldi-n-propylamine, dimethylbutylamine, trioctylamine and the like. In particular, the trialkylamine used in the organic extracting medium according to any aspect of the present invention may not be soluble in water and may be trioctylamine.

In one example, the organic extracting medium according to any aspect of the present invention may be a combination of alkyl-phosphine oxide or trialkylamine and at least one alkane. In particular, the alkane may comprise at least 12 carbon atoms. In particular, the alkane may comprise at 12-18 carbon atoms. In one example, the alkane may be selected from the group consisting of dodecane, tridecane, tetradecane, pentadecane, hexadecane, heptadecane and octadecane. In a further example, the organic extracting medium may comprise a mixture of alkanes. More in particular, the organic extracting medium according to any aspect of the present invention may be a combination of TOPO and tetradecane or hexadecane.

Trioctylphosphine oxide (TOPO) is an organophosphorus compound with the formula OP(C₈H₁₇)₃. TOPO may be part of the organic extracting medium together with at least one alkane according to any aspect of the present invention. In particular, the mixture of TOPO and alkane comprising at least 12 carbon atoms may comprise about 1:100 to 1:10 weight ratio of TOPO relative to the alkane. More in particular, the weight ratio of TOPO to alkane in the organic extracting medium according to any aspect of the present invention may be about 1:100, 1:90, 1:80, 1:70, 1:60, 1:50, 1:40, 1:30, 1:25, 1:20, 1:15, or 1:10. Even more in particular, the weight ratio of TOPO to alkane may be selected within the range of 1:90 to 1:10, 1:80 to 1:10, 1:70 to 1:10, 1:60 to 1:10, 1:50 to 1:10, 1:40 to 1:10, 1:30 to 1:10 or 1:20 to 1:10. The weight ratio of TOPO to alkane may be between 1:40 to 1:15 or 1:25 to 1:15. In one example, the weight ratio of TOPO to alkane may be about 1:15. In the example, the alkane may be hexadecane and therefore the weight ratio of TOPO to hexadecane may be about 1:15.

In another example, when the organic extracting medium comprises an alkyl-phosphine oxide or a trialkylamine that is more soluble in the alkane used in the organic extracting medium compared to the solubility of TOPO in alkane comprising at least 12 carbon atoms, the weight ratio of the alkyl-phosphine oxide (other than TOPO) or a trialkylamine to alkane may be 1:1, 2:1, 3:1, 4:1, 5:1, 6:1, 7:1, 8:1, 9:1 or 10:1. In one example, the organic extracting medium may be trihexy-phosphine oxide and the ratio of trihexy-phosphine oxide to alkane may be 1:1. In other examples, the organic extracting medium may be a lower chain alkyl-phosphine oxide and the ratio of the lower chain alkyl-phosphine oxide to alkane may be 2:1, 3:1, 4:1, 5:1, 6:1, 7:1, 8:1, 9:1 or 10:1. In this case, a lower-chain alkyl-phosphine oxide refers to a phosphine oxide with a C1-C4 alkyl group. In another example, the organic extracting medium may be a trialkylamine, this is known to be more soluble than phosphine oxide in alkanes. For example, the trialkylamine may be a trioctylamine (TOA) that may be present in the organic extracting medium according to any aspect of the present invention in the ratio of up to 1:1 with the alkane. Lower chain length amines can be used in even higher ratios. In other examples, the organic extracting medium may be a lower chain trialkylamine and the ratio of the lower chain trialkylamine to alkane may be 2:1, 3:1, 4:1, 5:1, 6:1, 7:1, 8:1, 9:1 or 10:1. In this case, a lower-chain alkyl-phosphine oxide refers to a phosphine oxide with a C1-C4 alkyl group.

The term 'about' as used herein refers to a variation within 20 percent. In particular, the term "about" as used herein refers to +/- 20%, more in particular, +/-10%, even more in particular, +/-5% of a given measurement or value.

As organic extracting medium in step e) the organic extracting medium alternatively preferably is selected from alkyl-phosphines oxides and trialkylamines, wherein the total amount of alkyl-phosphines oxides and trialkylamines referring to the total organic extraction medium is at least 98.0 wt.-%, preferably at least 99.5 wt.%

In step e) according to any aspect of the present invention, the organic acid and/or alcohol in the aqueous production medium may contact the organic extracting medium for a time sufficient to extract the organic acid and/or alcohol from the aqueous production medium into the organic extracting medium. A skilled person may be capable of determining the amount of time needed to reach distribution equilibrium and the right bubble agglomeration that may be needed to optimize the extraction process. In some examples the time needed may be dependent on the amount of organic acid and/or alcohol that may be extracted. In particular, the time needed to extract the organic acid and/or alcohol from the aqueous production medium into the organic extracting medium may only take a few minutes. According to any aspect of the present invention, where the extraction is carried out in step e) as production takes place in step d), the time for extraction may be equivalent to the time of production.

The ratio of the organic extracting medium used to the amount of organic acid and/or alcohol to be extracted may vary depending on how quick the extraction is to be carried out. In one example, the amount of organic extracting medium is equal to the amount of aqueous production medium comprising the organic acid and/or alcohol.

After the step of contacting the organic extracting medium with the aqueous production medium, the two phases (aqueous and organic) are separated using any means known in the art. This is schematically depicted as (4) and (5) in figure 2. In one example, the two phases may be separated using a separation funnel. The two phases may also be separated using mixer-settlers, pulsed columns, thermal separation and the like. In one example, where the organic acid is hexanoic acid, the separation of the organic extracting medium from the hexanoic acid may be carried out using distillation in view of the fact that hexanoic acid distills at a significantly lower boiling point than the organic extracting medium. A skilled person may be able to select the best method of separating the absorbent from the desired organic acid and/or alcohol depending on the characteristics of the organic acid and/or alcohol desired to be recovered.

In a preferred method according to the instant invention the organic acid and/or alcohol produced in step d) is acetic acid and/or ethanol, preferably with the acetogenic cell being *Clostridium autoethanogenum,* comprises a further step
f) contacting the acetic acid and/or ethanol with a second organism capable of converting acetic acid and/or ethanol to at least one fatty acid.

In this context the second organism preferably is *Clostridium kluyveri* and preferably the at least one fatty acid is hexanoic acid.

The fatty acid produced in step f) can be recovered in the same way as the organic acid in step e); thus preferably the method according to the invention contains the steps a) to d), f) and e) in the aforementioned order.

A preferred method according to the instant invention is a continuous process. Preferably in this continuous process the basic, aqueous medium depleted of the captured carbon dioxide from step c) is fed to step a) as a basic, aqueous medium. Even more preferably in step e) an organic extracting agent is used for recovering the organic acid and/or alcohol and the organic extracting agent is run in circles while being contained in the acidic, aqueous solution of the acidic compartment of the bipolar membrane electrodialysis unit in step c) as well as in the aqueous production medium in step d).

A very preferred method according to the instant invention is schematically depicted in figure 2).

It comprises the steps
a) providing a mixture of gases comprising carbon dioxide and contacting the mixture of gases with a basic, aqueous medium containing at least one base, thereby providing a basic, aqueous medium containing captured carbon dioxide in the form of hydrogencarbonate-anions and/or carbonate-anions by ;
b) feeding the basic, aqueous medium containing the captured carbon dioxide into a bipolar membrane electrodialysis cell comprising
   an anode,
   a cathode,
   an ion-selective anion-exchange membrane, and
   two water-dissociating bipolar membranes,
   capable of providing for an acidic compartment between the water-dissociating bipolar membrane on the anode side and the ion-selective anion-exchange membrane and for a basic compartment between the water-dissociating bipolar membrane on the cathode side and the ion-selective anion-exchange membrane;
c) regenerating the captured carbon dioxide to regenerated carbon dioxide by transporting the captured carbon dioxide in the form of hydrogencarbonate-anions and/or carbonate-anions across the anion-exchange membrane from the basic compartment into the acidic compartment in acidic, aqueous solution;
d) contacting the regenerated carbon dioxide and hydrogen with at least one acetogenic cell in an aqueous production medium in a fermenter comprising the at least one acetogenic cell, the aqueous production medium, a means of receiving a hydrogen stream, and a means of receiving the recovered carbon dioxide under suitable conditions to produce at least one organic acid and/or alcohol selected from acetic acid and ethanol from the regenerated carbon dioxide;
f) contacting the acetic acid and/or ethanol with a second organism capable of converting acetic acid and/or ethanol to at least one fatty acid; and
e) recovering the organic acid, which is fatty acid of step f).

This preferred method according to the instant invention preferably is further characterized by keeping the pH value within the bipolar membrane electrodialysis unit of the acidic compartment maintained by using the organic acid produced in step d) and not totally used in step f) or recovered in step e).

This preferred method according to the instant invention preferably is further characterized by keeping the pH value within the bipolar membrane electrodialysis unit of the basic compartment maintained by capturing the carbon dioxide in step a) in the basic, aqueous medium stemming from the basic compartment of the bipolar membrane electrodialysis unit and re-feeding it into the basic compartment.

This preferred method allows for an efficient continuous process.

Another aspect of the instant invention is an apparatus for performing a process according to the instant invention comprising:
a bipolar membrane electrodialysis unit comprising
an anode,
a cathode,
an ion-selective anion-exchange membrane,
a means of receiving captured carbon dioxide, and
two water-dissociating bipolar membranes,
capable of providing for an acidic compartment between the water-dissociating bipolar membrane on the anode side and the ion-selective anion-exchange membrane and for a basic compartment between the water-dissociating bipolar membrane on the cathode side and the ion-selective anion-exchange membrane;
a fermenter comprising
at least one acetogenic cell,
an aqueous production medium,
a means of receiving a hydrogen stream, and
a means of receiving the recovered carbon dioxide,
wherein the acetogenic cell is capable of converting the hydrogen and the regenerated carbon dioxide to at least one organic acid and/or alcohol.

Preferably the apparatus of the instant invention further comprises an ion-selective cation exchange membrane, that separates the acidic compartment from the anode and the basic compartment from the cathode. Preferably the ion-selective cation exchange membrane is Neosepta C66-10F supplied by Ameridia Corp.

Preferably the apparatus of the instant invention further comprises a means of contacting carbon dioxide in a mixture of gases with a basic, aqueous medium to capture the carbon dioxide.

Preferably the apparatus of the instant invention further comprises a means of recovering the organic acid and/or alcohol.

More preferably the apparatus of the instant invention further comprises a means for refeeding a portion of the organic acid and/or alcohol into the acidic compartment of bipolar membrane electrodialysis unit.

More preferably the apparatus of the instant invention further comprises a means for feeding a portion of the basic, aqueous medium from the basic compartment of bipolar membrane electrodialysis unit to the means of contacting carbon dioxide in a mixture of gases with a basic, aqueous medium.

Another aspect of the instant invention is the use of the apparatus according to the invention for performing the process according to the invention.

### Brief description of figures

Figure 1 is a schematic view of a bipolar membrane electrodialysis unit.
Figure 2 is a schematic view of an exemplary process of the instant invention
Figure 3 is a schematic view of the exemplary process of example 1 of the instant invention

The present invention is illustratively described in the examples listed below without any intention of limiting the invention, whose scope is determined by the entire description and the claims, to the embodiments referred to in the examples.

### Examples:

### Example 1:

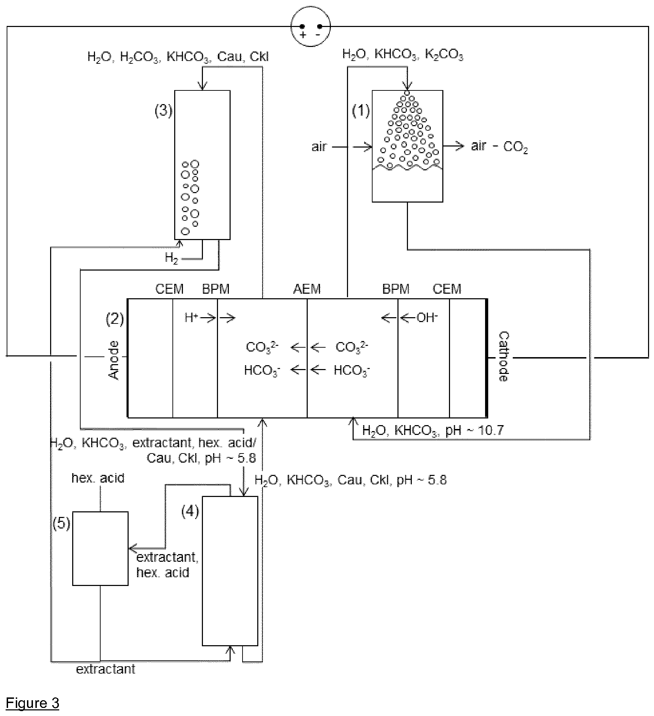

Air is scrubbed ((1) in figure 3) by an aqueous solution of KHCO₃ at a concentration of 0.485 mol/l, that also contains 0,008mol/l of K₂CO₃. The K₂CO₃ is converted by the absorbed CO₂ from air into KHCO₃ and therefore an aqueous solution with a total concentration of c=0.5 mol/l of KHCO₃ with less than0,001 mol/l K₂CO₃ and a pH of 10.7 is fed with a mass flow of 140 kg/h into the basic compartment of a bipolar membrane electrodialysis unit (2).

The bipolar membrane electrodialysis unit is a EUR2C-7-Bip assembled in the two-compartment configuration, (Ameridia Corp). It consists of seven cells in series. Each cell contains an ion-selective anion-exchange membrane of 200 cm² (Neosepta AHA supplied by Ameridia Corp.) in series with a water-dissociating bipolar membrane (Neosepta BP-1E supplied by Ameridia Corp.). At each end of the stack of seven cells is a cation exchange membrane (CEM in figure3, Neosepta C66-10F supplied by Ameridia Corp.) separating the cells from the electrode compartment. The spaces between adjacent membranes are filled with 762 mm thick polyethylene mesh spacers. The acidic compartment of the bipolar membrane electrodialysis unit contains the fermentation medium EvoDM39, see below.

The bipolar membrane electrodialysis unit is run with an amperage of 8 A and a voltage of 16 V. A mass flow of 14.0 m³/h from the acidic compartment of the bipolar membrane electrodialysis unit (2) is fed to a fermenter (3).

The fermenter (3) is a stirred tank reactor (rotation rate=500 rpm) with a volume of 1501. It is filled with 1101 of EvoDM39 medium (pH 5.8; 0.429 g/L Mg-acetate, 0.164 g/l Na-acetate, 0.016 g/L Ca-acetate, 2.454 g/l K-acetate, 0.107 mL/L H3PO4 (8.5%), 1.01 mL/L acetic acid, 0.35 mg/L Co-acetate, 1.245 mg/L Ni-acetate, 20 µg/L d-biotin, 20 µg/L folic acid, 10 µg/L pyridoxine-HCI, 50 µg/L thiamine-HCI, 50 µg/L Riboflavin, 50 µg/L nicotinic acid, 50 µg/L Ca-pantothenate, 50 µg/L Vitamin B12, 50 µg/L p-aminobenzoate, 50 µg/L lipoic acid, 0.702 mg/L (NH4)2Fe(SO4)2 x 4 H2O, 1 ml/L KS-acetate (93,5 mM)). The solution is neutralized with NH₃ to set the pH=5.8. The pressure is 1.0 bar

The vessel is inoculated with *Clostridium autoethanogenum (DSM 10061)* (Cau in figure 3) and *Clostridium kluyveri* (Ckl in figure 3) cells. After a growth phase of 48h at T=37 °C and the addition of a hydrogen volume flow of 150 Nl/h at a pressure of 1.1 bar an OD=2.4 with roughly the same biomass weight of *Clostridium autoethanogenum* and *Clostridium kluyveri* cells is achieved. 99% of the aqueous flow from the fermenter is recycled to the acidic compartment of the bipolar membrane electrodialysis unit. 1 % of the flow was fed into an extractor (4).

An additional flow of 2kg/h of an extractant, a mixture of 6% (w/w) TOPO in tetradecane, is fed to the extractor (4). The extractor is a stirred tank (r=100 rpm) with a residence time of 5 min and a continuously operated settler (V=10l). The total flow into the extractor is 142 kg/h. The flow is separated into one organic flow of 2 kg/h and one aqueous flow of 140 kg/h. The aqueous flow is recycled to the acidic compartment of the bipolar membrane electrodialysis unit.

The concentration of hexanoic acid in the extractant, the organic flow, is 19 g/kg. The determination of the product concentrations is performed by quantitative 1H-NMR spectroscopy. As an internal quantification standard sodium trimethylsilylpropionate (T(M)SP) is used.

Hexanoic acid is separated by distillation (5) and the deloaded extractant is recycled to the extractor (4).

## Claims

1. A method for production of organic acid and/or alcohol comprising the steps:
a) providing a basic, aqueous medium containing captured carbon dioxide in the form of hydrogencarbonate-anions and/or carbonate-anions;
b) feeding the basic, aqueous medium containing the captured carbon dioxide into a bipolar membrane electrodialysis unit comprising
an anode,
a cathode,
an ion-selective anion-exchange membrane, and
two water-dissociating bipolar membranes,
capable of providing for an acidic compartment between the water-dissociating bipolar membrane on the anode side and the ion-selective anion-exchange membrane and for a basic compartment between the water-dissociating bipolar membrane on the cathode side and the ion-selective anion-exchange membrane;
c) regenerating the captured carbon dioxide to regenerated carbon dioxide by transporting the captured carbon dioxide in the form of hydrogencarbonate-anions and/or carbonate-anions across the anion-exchange membrane from the basic compartment into the acidic compartment in acidic, aqueous solution;
d) contacting the regenerated carbon dioxide and hydrogen with at least one acetogenic cell in an aqueous production medium under suitable conditions to produce at least one organic acid and/or alcohol from the regenerated carbon dioxide; and optionally
e) recovering the organic acid and/or alcohol.

2. The method according to claim 1, wherein step a) comprises
providing a mixture of gases comprising carbon dioxide and contacting the mixture of gases with a basic, aqueous medium containing at least one base, thereby capturing the carbon dioxide, which is contained in the basic, aqueous medium in the form of hydrogencarbonate- and/or carbonate-anions.

3. The method according to claim 2, wherein the mixture of gases comprises carbon dioxide at a concentration of 350ppm-450ppm, preferably the mixture of gases is air.

4. The method according to either claim 2 or 3, wherein the basic, aqueous medium in step a) contains a base selected from the group of hydroxides and carbonates, preferably of alkaline and earth alkaline hydroxides and carbonates, preferably from the group of sodium hydroxide, potassium hydroxide and calcium hydroxide.

5. The method according to any one of the claims 2 to 4, wherein in step a) the basic, aqueous medium is sprayed through a nozzle into a stream of the mixture of gases comprising carbon dioxide.

6. The method according to any one of the preceding claims, wherein in step b) the bipolar membrane electrodialysis unit comprises an alternating stack of ion-selective anion-exchange membranes and water-dissociating bipolar membranes of an amount of from 3 to 20 membranes.

7. The method according to any one of the preceding claims, wherein in step c) an amperage of 5 A to 20 A, preferably 5 A to 15A most preferred 5 A to 10 A is applied to the bipolar membrane electrodialysis unit.

8. The method according to any one of the preceding claims, wherein in step d) the contacting takes place in a fermenter comprising the at least one acetogenic cell, the aqueous production medium, a means of receiving a hydrogen stream, and a means of receiving the recovered carbon dioxide.

9. The method according to any one of the preceding claims, wherein in step d) the pH of the aqueous production medium is in the range between 5.0 and 6.5.

10. The method according to any one of the preceding claims, wherein the organic acid and/or alcohol produced in step d) is acetic acid and/or ethanol.

11. The method according to any one of the preceding claims, wherein in step d) the acetogenic cell is selected from the group consisting of *Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium ljungdahlii (DSM 13528), Clostridium ljungdahlii C-01 (ATCC 55988), Clostridium ljungdahlii ERI-2 (ATCC 55380), Clostridium ljungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757),* and *Clostridium species ATCC 29797.*

12. The method according to any one of the preceding claims, wherein the organic acid and/or alcohol produced in step d) is acetic acid and/or ethanol, preferably with the acetogenic cell being *Clostridium autoethanogenum,* comprising a further step
f) contacting the acetic acid and/or ethanol with a second organism capable of converting acetic acid and/or ethanol to at least one fatty acid.

13. The method according to claim 12, wherein the second organism is *Clostridium kluyveri* and preferably the at least one fatty acid is hexanoic acid.

14. Apparatus for performing a process according to at least one of the claims 1 to 13 comprising:
a bipolar membrane electrodialysis unit comprising
an anode,
a cathode,
an ion-selective anion-exchange membrane,
a means of receiving captured carbon dioxide, and
two water-dissociating bipolar membranes,
capable of providing for an acidic compartment between the water-dissociating bipolar membrane on the anode side and the ion-selective anion-exchange membrane and for a basic compartment between the water-dissociating bipolar membrane on the cathode side and the ion-selective anion-exchange membrane;
a fermenter comprising
at least one acetogenic cell,
an aqueous production medium,
a means of receiving a hydrogen stream, and
a means of receiving the recovered carbon dioxide,
wherein the acetogenic cell is capable of converting the hydrogen and the regenerated carbon dioxide to at least one organic acid and/or alcohol.

15. Apparatus according to claim 14 further comprising a means of contacting carbon dioxide in a mixture of gases with a basic, aqueous medium to capture the carbon dioxide.

16. Apparatus according to claim 14 or 15 further comprising a means of recovering the organic acid and/or alcohol.

17. Use of the apparatus according to any one of claims 14 to 16, for performing the process according to any one of claims 1 to 13.
